# EUROPEAN PATENT APPLICATION

(11) **EP 3 272 264 A1**
(43) Date of publication of application: **24.01.2018**
(21) Application number: 15885562.7
(22) Date of filing: 06.11.2015
(51) Int. Cl.: A61B 1/00, G02B 23/24

(54) **ENDOSCOPE ATTACHMENT**

(30) Priority: 18.03.2015 JP 2015054976
(71) Applicant: Olympus Corporation, Tokyo 192-8507 (JP)
(72) Inventor: AIKAWA Yoshie, Tokyo 192-8507 (JP); MIKKAICHI Takayasu, Tokyo 192-8507 (JP)
(74) Representative: Schicker, Silvia
(86) International application number: PCT/JP2015/081293
(87) International publication number: WO 2016/147470

(57) **Abstract**

An endoscope attachment of the present invention includes a light-shielding member disposed in a distal direction from a distal end of an endoscope insertion portion of an endoscope and shields a part of light emitted from a light source of the endoscope at a position separated from the light source; and a support portion connected to the endoscope insertion portion and being capable of holding the light-shielding member at a position distant from the distal end of the endoscope insertion portion.

## Description

### [Technical Field]

The present invention relates to an endoscope attachment, and more particularly, to an endoscope attachment used for marking a desired position of a liminal tissue. Priority is claimed on Japanese Patent Application No. 2015-054976, filed March 18, 2015, the content of which is incorporated herein by reference.

### [Background Art]

Conventionally, in a treatment of an early malignant tumor or the like, for example, a procedure such as EMR (Endoscopic Mucosal Resection) and ESD (Endoscopic Submucosa Dissection) that endoscopically resects a lesion occurring in a mucosa inside a luminal organ such as the alimentary canal is performed. When the lesion infiltrates to a part deeper than a submucosal layer, a wide range of resection is surgically performed in many cases in consideration of the metastasis of the tumor. Although surgical resection is highly invasive and the QOL (Quality Of Life) of a patient is greatly impaired, the surgical resection includes some cases having no fear of metastasis.

Recently, local resection of lesions is beginning to be considered from the viewpoint that an optimal treatment should be performed for a patient. Since a position of a lesion is difficult to be specified from the side of an abdominal cavity when the lesion is locally resected in a laparotomy or laparoscopic surgery, the lesion must be resected with a large margin.

Therefore, a procedure that identifies a lesion position by an endoscope and indicates a certain region of a luminal organ including a margin from the inside of the luminal organ, and resects an optimal area from the side of the abdominal cavity, that is, from the outside of the luminal organ has been attempted. In the case of resection from the side of the abdominal cavity, since the target lesion is impossible to be directly observed the target lesion, an operation of indicating the target lesion from the inside of the luminal organ by the endoscope so that the target lesion can be seen from the side of the abdominal cavity is performed. For example, when an endoscope is used to indicate a resection area in the luminal organ or is used to perform marking so that the marking can be visually recognized from the side of the abdominal cavity, the resection area is specified by this marking.

Several related arts for indicating a specific position from the inside of a luminal organ so as to be visible from the side of an abdominal cavity are known. For example, there are several methods such as a method of locally injecting ink into a submucosal layer at a specific position and a method of pushing a specific position at a tip of an endoscope treatment instrument such as a pair of forceps or a high-frequency knife to cause the specific position to protrude on the abdominal cavity side. In addition, a method of observing a lesion from the side of the abdominal cavity by emitting light having a focused luminous flux from the inside of the luminal organ toward a specific position (see, for example, Patent Document 1) or a method of indwelling a metallic coil or a tag to a specific position to penetrate the luminal organ is also known.

### [Prior Art Documents]

### [Patent Document]

[Patent Document 1]
US Patent No. 7273451 specification

### [Summary of the Invention]

### [Problems to be Solved by the Invention]

There are several issues and points to pay attention to when performing the above-described marking.

First, in the above-described methods using an ink or a pair of forceps of an endoscope, a position on the mucosal side cannot be indicated precisely toward the abdominal cavity side. In the method using an ink, an area which is dyed with the ink is widened. In the method using a pair of forceps, an indication range varies depending on the sense of an operator.

Next, a "layer shift" in a wall of a luminal organ exists. For example, in a tissue of a stomach wall, a mucosal layer from an innermost surface to a submucosal layer and a muscle layer portion from a muscle layer to a serous membrane are loosely connected via a connective tissue of a boundary portion and the like. Therefore, the mucosal layer and the mucosal layer portion move relative to each other in a plane direction orthogonal to a thickness direction of the wall so that a layer shift easily occurs. Thus, for example, in the method of pushing a lesion by a tip of an endoscope treatment instrument, a mucosal layer portion located at a specific position may be different between when the specific position is caused to protrude and after the protrusion is released. As a result, there is a problem in which precise resection cannot be performed from the abdominal cavity side. In addition, even when the indication is performed by light as in Patent Literature 1, a marking position on the mucosal side and a marking position on the abdominal cavity side may eventually be displaced from each other. If the marking positions are different from each other, there is a possibility that a lesion may be resected at a position closer to the tumor inside the margin. As a result, there is a concern that cancer cells may remain.

Next, contamination inside an abdominal cavity can occur. Since tags such as T bars are indwelled to penetrate a wall of a luminal organ, no layer shift occurs after the indwelling. However, if the tags are indwelled inside the luminal organ, there is a possibility that contents of the luminal organ may move into the abdominal cavity. In a case in which a lesion is a malignant tumor, if tumor cells are contained in the contents, the tumor cells may grow inside the abdominal cavity. For this reason, it is desirable to reduce or completely remove such a risk.

Furthermore, since a plurality of devices such as an endoscope and a treatment instrument are used in a procedure such as EMR or ESD, there is also a desire for miniaturizing devices to be used and saving power used by the devices.

However, none of the above-described related arts satisfy all the above-described points, and a device capable of performing more preferable marking is required.

In view of the above-described circumstances, an object of the present invention is to provide an endoscope attachment capable of appropriately marking a specific position of a luminal organ.

### [Means for Solving the Problem]

An endoscope attachment according to a first aspect of the present invention includes: a light-shielding member disposed in a distal direction from a distal end of an endoscope insertion portion of an endoscope and shields a part of light emitted from a light source of the endoscope at a position distally apart from the light source; and a support portion connected to the endoscope insertion portion and being capable of holding the light-shielding member at a position distant from the distal end of the endoscope insertion portion.

A second aspect of the present invention provides the endoscope attachment according to the first aspect, may further include: an operation member connected to the light-shielding member, provided to extend to an operation portion of the endoscope, and switches between a light-shielded state and a light-shielding-released state, the light-shielded state by moving the light-shielding member, the light-shielded state in which the light-shielding member is disposed at a position on a distal extension line of the light source, the light-shielding-released state in which the light-shielded state is released.

A third aspect of the present invention provides the endoscope attachment according to the first or second aspect, wherein the light-shielding member may include a light-shielding surface covering a distal end surface of the endoscope insertion portion at position distant from the distal end surface of the endoscope insertion portion. An open portion transmitting transmission of light in an optical axis direction of the light source may be formed at a part of the light-shielding surface.

A fourth aspect of the present invention provides the endoscope attachment according to any one of the first to third aspects, wherein the support portion may be a cylindrical member being fittable onto the distal end part of the endoscope insertion portion.

A fifth aspect of the present invention provides the endoscope attachment according to the fourth aspect, wherein the light-shielding member may be a lid-shaped member disposed at a distal end of the support portion, and wherein the light-shielding member may be provided to be detachable from the distal end of the support portion.

A sixth aspect of the present invention provides the endoscope attachment according to the fourth or fifth aspect, wherein the support portion may include a light-transmitting portion, which is capable of transmitting light, on a side surface parallel to a longitudinal axis.

A seventh aspect of the present invention provides the endoscope attachment according to the third aspect, wherein the light-shielding member may be formed by a stretchable covering member having a light-shielding property and capable of covering the support portion, and wherein the light-shielding member may cover the support portion so as to be capable of forming an open portion having an opening diameter smaller than an outer peripheral diameter of the endoscope insertion portion at a distal end of the support portion and the light-shielding member may make transmission of light only from the open portion at a position distant from the distal end of the endoscope insertion portion.

An eighth aspect of the present invention provides the endoscope attachment according to the first or second aspect, wherein the support portion may include a plurality of elastic arm portions having a restoration force to restore each of the arm portions to a predetermined curved shape, wherein the light-shielding member may be formed by a stretchable film having a light-shielding property. The light-shielding member may be configured to be formed such that an open portion having an area smaller than that of a distal end surface of the endoscope insertion portion is formed, an outer peripheral edge is fixed to distal ends of the plurality of arm portions, and a light-shielding surface is capable of being formed on a plane including the distal ends of the plurality of arm portions, wherein the light-shielding member and the plurality of arm portions may be formed in a deformable manner to be inserted into a sheath, which is inserted through a channel formed along a longitudinal axis direction of the endoscope insertion portion, and to be capable of advancing and retracting from a distal end of the sheath, and wherein the distal ends of the arm portions are configured to curve to be away from each other when the light-shielding member and the arm portions may protrude from the distal end of the sheath, and the light-shielding member may form the light-shielding surface at a position distant from the distal end of the endoscope insertion portion and may shield light in a periphery of the open portion.

A ninth aspect of the present invention provides the endoscope attachment according to the third aspect, wherein the light-shielding member may be a lid-shaped member disposed at a distal end of the support portion and is provided with the open portion, and wherein the light-shielding member may include a light-shielding area varying mechanism that capable of changing a size of a light-shielding area in a periphery of the open portion.

A tenth aspect of the present invention provides the endoscope attachment according to the third aspect, wherein the light-shielding member may include: a first polarizing filter disposed at a distal end of the support portion and is provided with the open portion, a second polarizing filter disposed adjacent to the light source and at a proximal end of the support portion, and a movement mechanism that rotates the first polarizing filter in a circumferential direction of the support portion. The light-shielding member may be configured to be capable of shielding in a periphery of the open portion by the movement mechanism that changes a polarizing angle with respect to the second polarizing filter by rotating the first polarizing filter.

### [Advantageous Effects of Invention]

According to the endoscope attachment of the present invention, it is possible to appropriately mark a specific position of a luminal organ so that the mark is visible from the inside and the outside of the luminal organ by attaching the endoscope attachment to an existing endoscope apparatus.

### [Brief Description of Drawings]

Fig. 1 is a perspective view showing an endoscope attachment according to a first embodiment of the present invention.
Fig. 2 is a cross-sectional view showing a state in which the endoscope attachment according to the first embodiment of the present invention is mounted on an endoscope insertion portion.
Fig. 3 is a cross-sectional view showing a light-shielding-released state of the endoscope attachment according to the first embodiment of the present invention.
Fig. 4A is a diagram showing an aspect of usage of the endoscope attachment according to the first embodiment of the present invention.
Fig. 4B is a diagram showing an aspect of usage of the endoscope attachment according to the first embodiment of the present invention.
Fig. 4C is a diagram showing an aspect of usage of the endoscope attachment according to the first embodiment of the present invention.
Fig. 5 is a cross-sectional view showing a modified example of the first embodiment.
Fig. 6 is a perspective view showing an endoscope attachment according to a second embodiment of the present invention.
Fig. 7 is a cross-sectional view showing a state in which the endoscope attachment according to the second embodiment of the present invention is mounted on an endoscope insertion portion.
Fig. 8 is a cross-sectional view showing a light-shielding-released state of the endoscope attachment according to the second embodiment of the present invention.
Fig. 9 is a perspective view showing an endoscope attachment according to a third embodiment of the present invention.
Fig. 10 is a side view showing an aspect of usage of the endoscope attachment according to the third embodiment of the present invention.
Fig. 11 is a perspective view showing an aspect of usage of the endoscope attachment according to the third embodiment of the present invention.
Fig. 12 is a plan view showing a support portion of a modified example of the third embodiment.
Fig. 13 is a perspective view showing an endoscope attachment according to a fourth embodiment of the present invention.
Fig. 14 is a cross-sectional view showing an aspect of usage of the endoscope attachment according to the fourth embodiment of the present invention.
Fig. 15 is a perspective view showing an aspect of usage of the endoscope attachment according to the fourth embodiment of the present invention.
Fig. 16 is an exploded perspective view showing a light-shielding member of an endoscope attachment according to a fifth embodiment of the present invention.
Fig. 17 is a plan view showing a light-shielded state of the endoscope attachment according to the fifth embodiment of the present invention.
Fig. 18 is a cross-sectional view showing an endoscope attachment according to a sixth embodiment of the present invention.
Fig. 19 is a perspective view showing a state in which the endoscope attachment according to the sixth embodiment of the present invention is mounted on an endoscope insertion portion.

### [Description of Embodiments]

### [First Embodiment]

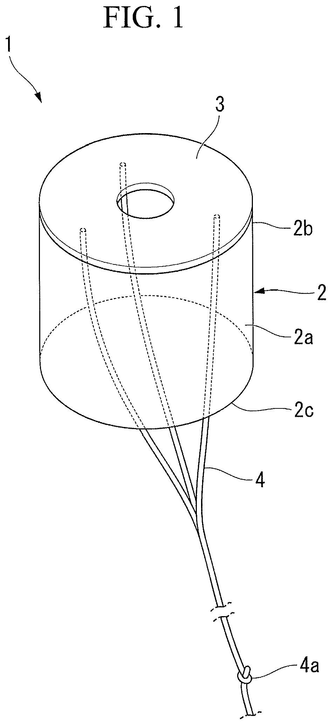

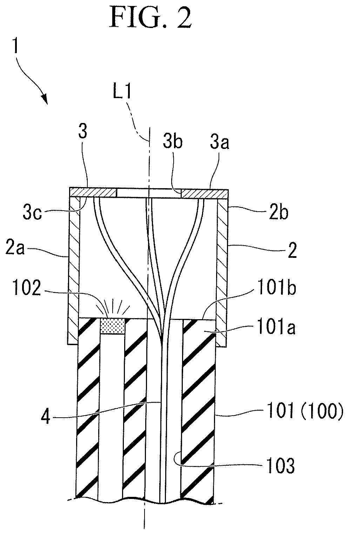

An endoscope attachment (hereinafter, simply referred to as an "attachment") according to a first embodiment of the present invention will be described. Fig. 1 is a perspective view showing an attachment 1 according to this embodiment. Fig. 2 is a cross-sectional view showing a state in which the attachment 1 is mounted on an endoscope insertion portion 101 of an endoscope apparatus 100.

The attachment 1 according to the embodiment is a member detachably attached to a distal end part 101a of the endoscope insertion portion 101 and is used while attached to a distal end of the endoscope insertion portion 101. As shown in Fig. 1, the attachment 1 includes a support portion 2 and a light-shielding member 3.

The support portion 2 is mounted on the distal end part 101a of the endoscope insertion portion 101. The support portion 2 is connected to the endoscope insertion portion 101 and is provided to keep the light-shielding member 3 at a position distant from the distal end part 101a of the endoscope insertion portion 101. Specifically, the support portion 2 is a cylindrical member which is capable of being fitted onto the distal end of the endoscope insertion portion 101. An inner diameter of the support portion 2 is substantially the same as an outer diameter of the distal end part 101a of the endoscope insertion portion 101. The support portion 2 includes a side wall portion 2a extending in an axial direction and opens at a distal end part 2b.

The side wall portion 2a of the support portion 2 is formed as a member capable of transmitting light through a side surface parallel to a longitudinal axis of the support portion 2. Thus, the side wall portion 2a is formed as a light-transmitting portion. Since the side wall portion 2a of the support portion 2 is formed as the light-transmitting portion, a field of view of the endoscope apparatus 100 is capable of being kept bright even when the attachment 1 is attached thereto.

The light-shielding member 3 is a disk-shaped member having a light-shielding property and having a size substantially the same as an outer peripheral diameter of the distal end part 2b of the support portion 2, and an open portion 3b is formed at a substantial center portion of the light-shielding member. In the light-shielding member 3, a periphery of the open portion 3b forms a light-shielding surface 3a. As shown in Fig. 2, the light-shielding member 3 is disposed in a distal direction from the distal end part 101a of the endoscope insertion portion 101. The light-shielding member 3 is disposed to cover the distal end part 2b of the support portion 2 with the light-shielding surface 3a. The light-shielding member 3 covers a distal end surface 101b of the endoscope insertion portion 101 by a position distally apart from the endoscope insertion portion 101 by the light-shielding surface 3 a which is the periphery of the open portion 3b when the attachment 1 is mounted on the endoscope insertion portion 101. The open portion 3b is formed at a part of the light-shielding surface 3a to pass through the light-shielding member 3 in a thickness direction of the light-shielding member 3, and transmits light in an optical axis direction of a light source 102. The light-shielding member 3 is formed as, for example, a thin plate having a light-shielding property, and is formed of stainless steel or a resin. For example, the light-shielding member may be also formed such that the light-shielding surface is formed by bonding a light-shielding film to a portion corresponding to a light-shielding surface of a thin plate which has a light-transmitting property and has the open portion 3b.

As shown in Fig. 2, the support portion 2 is mounted on the endoscope insertion portion 101 by fitting a proximal end 2c of the support portion 2 onto the outer periphery of the distal end part 101a of the endoscope insertion portion 101. The support portion 2 is formed to extend along a longitudinal axis L1 of the endoscope insertion portion 101 when the attachment 1 is mounted on the distal end part 101a of the endoscope insertion portion 101.

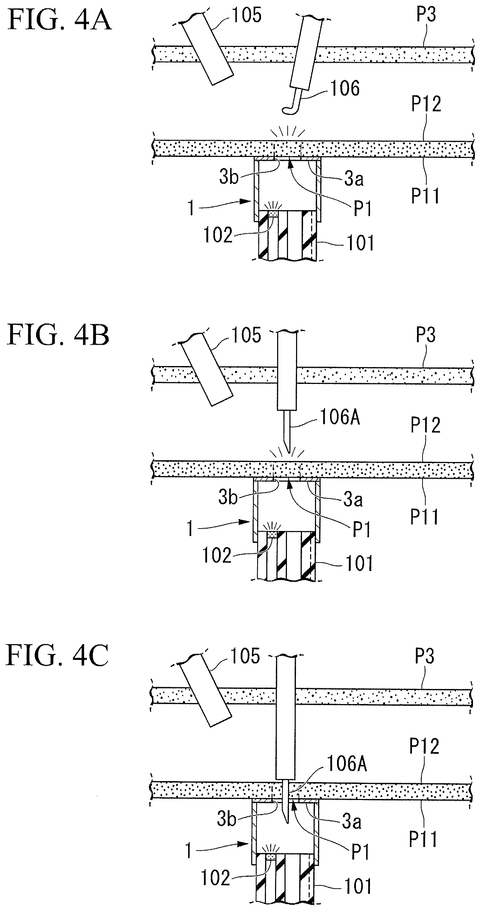

As shown in Fig. 2, in the endoscope apparatus 100, a light source 102 for illumination is provided at the distal end part 101a of the endoscope insertion portion 101. When the attachment 1 is mounted on the distal end part 101a of the endoscope insertion portion 101, the light-shielding member 3 is disposed at a position on a distal extension line of the light source 102 and shields a part of light that is emitted from the light source 102 provided in the endoscope apparatus 100 at a position distally apart from the light source 102. It is not necessary to dispose a position of the open portion 3b of the light-shielding member 3 on an optical axis of the light source 102. For example, the position of the open portion 3b may be set to a position relative to an image-capturing unit so that the position of the open portion 3b easily matches a marking position P1 (see Fig. 4A) while an image captured by the image-capturing unit is observed by an operator.

The light-shielding member 3 is provided to be movable from the distal end part 2b of the support portion 2. For the purpose of showing a marking position by shielding a part of the light emitted from the light source 102 of the endoscope apparatus 100, the light-shielding member 3 is enough to be fixed to a position in front of the endoscope insertion portion 101. However, since a procedure needs to be performed highly efficiently in a short time, it is desirable to be able to continuously perform other procedures after a marking process while the attachment 1 is attached to the endoscope insertion portion. Here, in the attachment 1 according to this embodiment, the light-shielding member 3 is provided to be movable relative to the support portion 2.

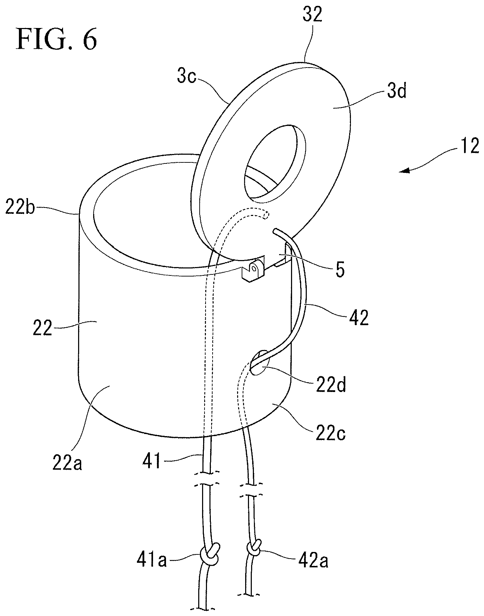

In the light-shielding member 3, a surface located on the side of the support portion 2 is set as a proximal surface 3c while the light-shielding member 3 is attached to cover the distal end part 2b of the support portion 2. Ends of a plurality of yarns 4 are fixed to the proximal surface 3c to be away from each other in a circumferential direction of the light-shielding member 3. In this embodiment, three yarns 4 are fixed to the proximal surface 3c of the light-shielding member to be away from each other at the same interval in the circumferential direction. The three yarns 4 are integrated into one yarn on the proximal side, and the one yarn is inserted into the side wall portion 2a of the support portion 2. As shown in Fig. 2, when the attachment 1 is mounted on the endoscope insertion portion 101, the proximal end side of the yarn 4 is inserted through a treatment instrument channel 103 formed in a longitudinal direction inside the endoscope insertion portion 101 and is inserted into an operation portion (not shown) provided at the proximal end part of the endoscope apparatus 100. A knot portion 4a (see Fig. 6) is provided at each of the proximal end side of the yarn 4. When the operator pulls the knot portion 4a exposed on the operation portion side toward the proximal end side, the light-shielding member 3 is pressed toward the distal end part 2b of the support portion 2 so as to be maintained a state that the light-shielding member 3 is disposed at the distal end part 2b of the support portion 2. Further, since the three yarns 4 are integrated as the one yarn at the proximal end side, rotation of the light-shielding member 3 about the axis is suppressed while the yarn 4 is pulled toward the proximal side so that the light-shielding member 3 can be stably supported while the light-shielding member 3 is in contact with the distal end part 2b of the support portion 2.

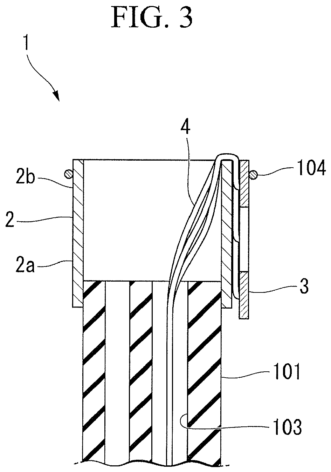

Fig. 3 is a cross-sectional view showing a light-shielding-released state of the attachment 1. As shown in Fig. 3, the light-shielding-released state is a state that the light-shielding member 3 is separated from the distal end part 2b of the support portion 2 and is disposed along the side wall portion 2a so that the side wall portion 2a and the light-shielding member 3 are fixed by a band 104 connected to the side wall portion 2a through a connection portion (not shown). The attachment in the light-shielding-released state as an initial state may be inserted into a body. In a light-shielding-released state in which the light-shielding member 3 is separated from the support portion 2, a distal direction of the endoscope insertion portion 101 is opened. As a result, a known treatment to a tissue is able to be performed by protruding a treatment instrument from the treatment instrument channel 103 formed in the endoscope insertion portion 101. At this time, as shown in Fig. 3, the plurality of yarns 4 pass through the side wall portion 2a of the support portion 2, are inserted through the treatment instrument channel 103 of the endoscope insertion portion 101, and are exposed at the operation portion side. For that reason, when it is necessary to attach the light-shielding member 3 to the distal end part 2b of the support portion 2 after a known treatment instrument is inserted from the treatment instrument channel 103 to perform an appropriate treatment, the operator pulls the proximal end part of the yarn 4 toward the proximal side so that the light-shielding member 3 is inclined with respect to the side wall portion 2a and the band 104 is moved in the distal direction of the attachment 1, thereby releasing a locking of the band 104. When the yarn 4 is further pulled, the light-shielding member 3 approaches the distal end part 2b of the support portion 2 to move onto the distal end part 2b of the support portion 2 as shown in Fig. 2 so that the position of the light-shielding member 3 is fixed as described above.

When the light-shielding member 3 placed on the support portion 2 is separated from the distal end part 2b of the support portion 2 again, the operator loosens the pulled yarn 4 or protrudes the treatment instrument (not shown) from the treatment instrument channel 103 so that the proximal surface 3c of the light-shielding member 3 is pressed in the distal direction. In this way, the light-shielding member 3 is able to be separated from the support portion 2. At this time, since the yarns 4 are fixed to the light-shielding member 3, the light-shielding member 3 is able to be reliably extracted from the inside of a body after the operation. Since the stretchable band (hereinafter, referred to as a "band") 104 such as a rubber band is connected to the side wall portion 2a, the band 104 is able to be reliably extracted from the inside of the body after the operation. The band 104 may not be fixed to the distal end part 2b. In this case, the separated band falls into the stomach and is discharged together with feces and the like.

Next, a procedure using the endoscope apparatus 100 to which the attachment 1 is attached will be described. Figs. 4A to 4C are diagrams showing aspects of usage of the attachment 1. In Figs. 4A to 4C, the yarn 4 is not shown. An operator inserts the endoscope insertion portion 101 having the attachment 1 attached thereto into a body so that the light-shielding member 3 is disposed in the vicinity of a marking position P1 of a tissue. At this time, the light-shielding member 3 is mounted on the distal end part 2b of the support portion 2.

First, the endoscope apparatus 100 performs marking (cauterization) at a predetermined position P1 of a first plane P11 of a tissue. The marking is performed at a plurality of positions P1 around a treatment target site.

Next, the operator specifies the marking position P1 by observing the first plane P11 of the tissue using an image-capturing unit (not shown) provided in the endoscope insertion portion 101. Subsequently, the operator positions the endoscope insertion portion 101 so that the marking position P1 is viewed through the open portion 3b formed in the light-shielding member 3. Then, the operator presses the light-shielding member 3 against the first plane P11 of the tissue. In this state, intensity of the light source 102 may be maximized. As a result, the light emitted from the light source 102 through the open portion 3b of the light-shielding member 3 is transmitted through the marking position P1. In this state, a second plane (an opposite plane) P12 of the tissue on a side opposite to the first plane (a proximal plane) P11, which is near the light-shielding member 3, of the marking position P1 is observed by using a laparoscope 105 inserted into a body cavity from an abdominal wall P3 while a light source of the laparoscope 105 is extinguished or weakened. As a result, the light along a contour shape of the open portion 3b is capable of seeing and specifying the marking position P1 from the second plane P12 by using the laparoscope 105. At this time, since the periphery of the open portion 3b is shielded by the light-shielding surface 3 a, a position of the transmitted light is capable of being clearly seen by a bright portion along the contour shape of the open portion 3b and a dark portion in the periphery thereof on the side of the second plane P12 of the tissue. That is, the light-shielding member 3 is capable of shielding a part of the light emitted from the light source 102 at a position distally apart from the light source 102 and allows the transmission of the light emitted from the light source 102 at a predetermined position.

Subsequently, marking is performed on the second plane P12 of the tissue. As shown in Fig. 4A, the operator performs marking by which a treatment instrument such as an electric scalpel 106 inserted from the abdominal wall P3 contacts the marking position P1 while checking a mark formed by the light transmitted to the second plane P12 of the tissue using the laparoscope 105 and cauterizes a tissue.

As a method of performing marking from the second plane P12 of the tissue, for example, as shown in Figs. 4B and 4C, a method of indwelling a tag using a tag-indwelling device is exemplified in addition to the method of cauterizing a second plane of a tissue using the electric scalpel 106. In the case of the method of indwelling a tag, it is possible to prevent an occurrence of a layer shift of the tissue.

Specifically, similarly to the method using the electric scalpel 106, a mark formed by the light transmitted on the second plane P12 of the tissue is punctured by a puncture needle 106A of the tag-indwelling device while being checked by the laparoscope 105. At this time, since the first plane P11 of the tissue in the periphery of the marking position P1 is supported by the light-shielding member 3 of the attachment 1 so that the first plane is pressed, the tissue is not easily displaced when the tissue is punctured by the puncture needle 106A. In addition, since the first plane P11 of the tissue in the periphery of the marking position P1 contacts with the light-shielding member 3 of the attachment 1, the tissue is supported on the side of the first plane P11 when the tissue is punctured by the puncture needle 106A from the second plane P12 of the tissue. Thereby, the puncture needle 106A is easily press-inserted into the tissue. Further, since the attachment 1 is configured such that the light-shielding member 3 is disposed at a position distant from the distal end surface 101b of the endoscope insertion portion 101, the endoscope apparatus 100 is not damaged when the treatment instrument such as the electric scalpel or the puncture needle punctures the tissue from the second plane P12 toward the first plane P11 of the tissue so as to penetrate the tissue. In addition, it is possible to observe a state in which the puncture needle 106A is inserted into the second plane P12 of the tissue by the endoscope apparatus 100.

When the tissue is punctured by the puncture needle 106A, a tag such as a T bar (not shown) is indwelled from a needle tip.

The marking process shown in Fig. 4A or Figs. 4B and 4C is repeatedly performed at several places around the treatment target site in order to specify a resected position of the treatment target site.

After the marking process is performed on all positions of the second plane P12 corresponding to a desired marking position using the electric scalpel 106 or the tag-indwelling device, the operator retracts the endoscope insertion portion so as to apart the attachment 1 from the first plane P11 of the tissue to the proximal side. At this time, the light-shielding member 3 may be separated from the support portion 2 in the above-described manner to be in the light-shielding-released state. Next, the operator extracts the endoscope insertion portion 101 to the outside of the body along with the attachment 1.

According to this embodiment, a configuration is simple because the attachment 1 is used while being mounted on the existing endoscope apparatus 100, and it is possible to perform marking on the second plane P12 of the tissue by using the light source provided in the endoscope apparatus 100. Since the light-shielding member 3 is disposed at a position distally apart from the distal end part 101a of the endoscope insertion portion 101, an original function of the endoscope apparatus 100 is not damaged.

In the attachment 1 according to this embodiment, when the tissue is punctured from the second plane P12 of the tissue by the treatment instrument such as the tag-indwelling device, the tissue is pressed so that the tissue is reliably punctured by a puncture needle of the tag-indwelling device. For that reason, the endoscope apparatus 100 is not damaged by the tag-indwelling device. Additionally, since the light-shielding member 3 is provided to be detachable from the distal end part 2b of the support portion 2, treatment is capable of being continuously performed before and after the marking.

In the attachment 1 according to this embodiment, since the light source 102 of the endoscope apparatus 100 can be used, there is no need to separately provide a light source for the marking, and thus a further cost reduction may be realized.

In this embodiment, an example in which the circular open portion 3b is formed at the substantial center of the light-shielding surface 3a of the light-shielding member 3 has been described, but an aspect of shielding light by the light-shielding member 3 is not limited thereto. Here, the marking position P1 of the tissue may be viewed as a bright portion from the second plane P12 of the tissue. For example, the position of the open portion is not limited to the substantial center of the light-shielding surface and may be formed at a desired position. Further, a light-shielding portion may be formed in the periphery of the open portion 3b. For example, a configuration in which a ring-shaped light-shielding film is bonded to the light-shielding surface of the light-shielding member 3 and light is transmitted through an inside and an outside of the ring of the light-shielding film may be employed. In this case, when the marking position P1 matches the inside of the ring, in the second plane P12, the marking position P1 is a bright portion, and a ring-shaped dark portion in the periphery thereof is capable of being seen so that this portion is recognized as a mark by the operator.

Additionally, a surface which shields light by the light-shielding member 3 may be the light-shielding surface 3a or the proximal surface 3c.

In this embodiment, an example in which the open portion 3b of the light-shielding member 3 is a through hole has been described, but the open portion 3b is not limited to being the through hole. For example, when marking is performed only on the second plane P12 while the electric scalpel 106 contacts with the surface of the second plane P12 of the marking position P1 of the tissue, the tissue is not punctured by the electric scalpel 106. In this case, the light-shielding member may have, for example, a configuration in which a film having an open portion and a light-shielding property is bonded to a desired position of a flat plate having a light-transmitting property to form the light-shielding surface 3a.

### [Modified Example]

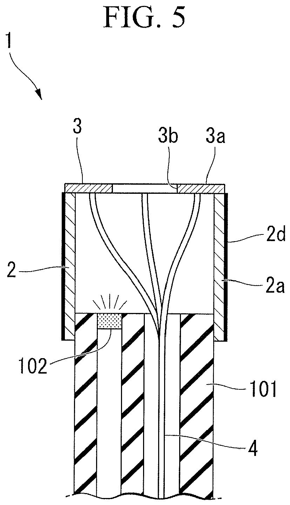

Fig. 5 shows a cross-sectional view of a modified example of the attachment 1 according to the embodiment. In the modified example shown in Fig. 5, a configuration of the support portion 2 is different from that of the first embodiment. That is, in the above-described embodiment, an example in which the light-transmitting portion making the transmission of light therethrough is provided on the side surface (the side wall portion 2a) parallel to the longitudinal axis of the support portion 2 has been described. However, in the modified example, the side wall portion 2a is covered by a light-shielding film 2d so that light is not transmitted therethrough. In this way, when the light is shielded by the side wall portion 2a of the support portion 2, a diffusion light diffused from the light source 102 of the endoscope apparatus 100 is prevented from leaking to an outer peripheral side of the side wall portion 2a of the attachment 1 so that the diffusion light is emitted only from the open portion 3b. As a result, the marking position is capable of more accurately checking by using the laparoscope 105.

### [Second Embodiment]

Next, a second embodiment of the present invention will be described with reference to Figs. 6 to 8. This embodiment is different from the first embodiment in that a connection structure between the light-shielding member and the support portion, and that a light-shielding member movement mechanism and an operation member are provided. In the following description, the same reference numerals are used for components and members which are the same as or similar to the components and members described in the above embodiment, and descriptions thereof are omitted.

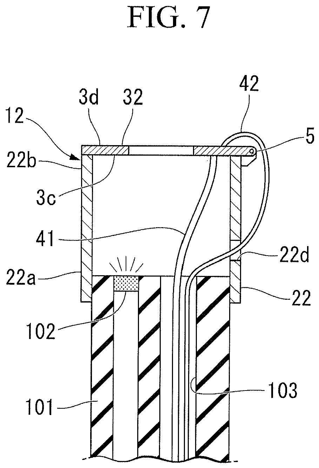

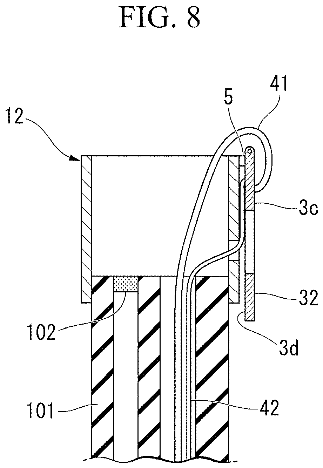

Fig. 6 is a perspective view showing an attachment 12 according to this embodiment. Fig. 7 is a perspective view showing a state in which the attachment 12 is mounted on the endoscope insertion portion 101. Fig. 8 is a cross-sectional view showing a light-shielding-released state of the attachment 12.

A light-shielding member 32 of the attachment 12 according to this embodiment is connected to a distal end part 22b of a support portion 22 through a hinge (a light-shielding member movement mechanism) 5. The light-shielding member 32 is provided in the support portion 22 to be openable and closable. That is, the attachment 12 is capable of being switched between a light-shielded state and a light-shielding-released state. The light-shielded state is a state that the light-shielding member 32 is moved by the hinge 5 so that the light-shielding member 32 is disposed on a distal side extension line from the light source 102. The light-shielding-released state is a state that the light-shielding member 3 is moved from the distal end part 22b of the support portion 22 so that the distal end part 22b of the support portion 22 is opened.

In the light-shielded state in which the light-shielding member 32 covers the distal end part 22b of the support portion 22, a surface on the proximal side of the light-shielding member 32 will be referred to as a proximal surface 3c and a surface on the distal side thereof will be referred to as a distal surface 3d. As shown in Figs. 6 and 7, a first yarn (an operation member) 41 is fixed to the proximal surface 3c, and a second yarn (an operation member) 42 is fixed to the distal surface 3d. The first yarn 41 and the second yarn 42 are used to switch between the light-shielded state and the light-shielding-released state by moving the light-shielding member 32. The first yarn 41 is inserted into an inside of a side wall portion 22a of the support portion 22. A proximal end part 22c of the support portion 22 is provided with a through hole 22d which communicates with the inside and an outside of the side wall portion 22a. The second yarn 42 is inserted through the through hole 22d from the outer peripheral side of the side wall portion 22a while a vicinity of a fixed portion with respect to the distal surface 3d is disposed at the outside of the side wall portion 22a. When the attachment 12 is mounted on the endoscope insertion portion 101, the first yarn 41 and the second yarn 42 are inserted through the treatment instrument channel 103 and are exposed at the operation portion side. Knot portions 41 a and 42a are respectively provided at the proximal end sides of the first yarn 41 and the second yarn 42, and the knot portions 41 a and 42a exposed at the operation portion side are pulled by an operator.

When the light-shielded state (Fig. 7) in which the light-shielding member 32 covers the support portion 22 is changed to the light-shielding-released state in which the light-shielding member 32 is opened, the operator pulls the second yarn 42 toward the proximal side. As a result, the light-shielding member 32 is pulled in a distal direction so that the light-shielding member 32 rotates about the hinge 5 to be in the light-shielding-released state. In the light-shielding-released state, the light-shielding member 32 rotates toward a position along the outer peripheral surface of the side wall portion 2a and disposed thereto as shown in Fig. 8.

When the light-shielding-released state is changed to the light-shielded state, the operator pulls the first yarn 41 toward the proximal side so that the light-shielding member 32 rotates about the hinge 5 in a direction approaching the distal end part 2b of the support portion 22. The first yarn 41 is pulled until the light-shielding member 32 is brought into contact with the distal end part 2b of the support portion 22.

Even though the second yarn 42 is not provided, if the treatment instrument is protruded from the treatment instrument channel 103 so that the proximal surface 3c is pressed in a distal direction, the light-shielding member 32 rotates about the hinge 5 so that the light-shielded state is changed to the light-shielding-released state similarly to the first embodiment.

When thicknesses or colors of the first yarn 41 and the second yarn 42 are changed, it is desirable in that the operator is possible to easily distinguish the first yarn 41 and the second yarn 42. In this embodiment, the first yarn 41 is thicker than the second yarn 42.

According to this embodiment, it is possible to show a marking position on the opposite plane (the second plane P12) of the tissue by using an endoscope illumination light source similarly to the above-described embodiment.

According to this embodiment, the light-shielding member 32 is easily moved relative to the support portion 2, and the light-shielded state and the light-shielding-released state is easily switched between. As a result, it is possible to repeatedly perform a series of procedures before and after the marking without separating the attachment 12.

### [Third Embodiment]

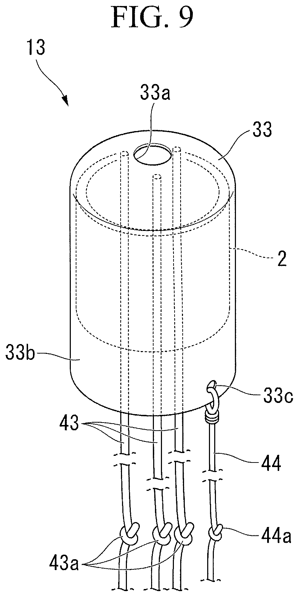

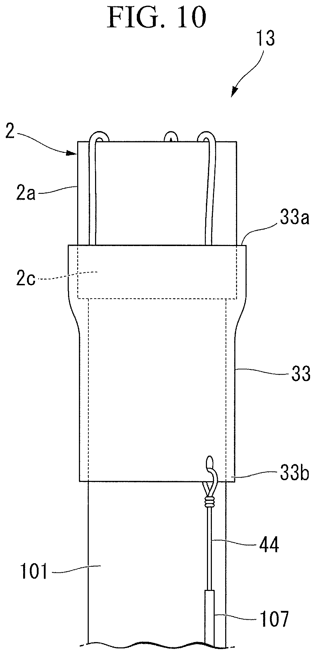

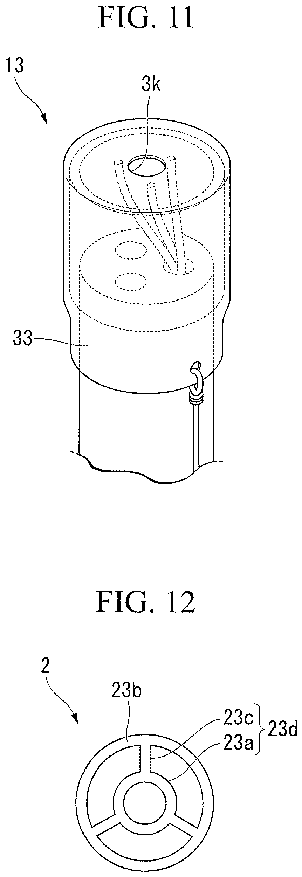

Next, a third embodiment of the present invention will be described with reference to Figs. 9 to 11. In this embodiment, the light-shielding member is different from those of the above-described embodiments. The support portion 2 is the same as that of the first embodiment. Fig. 9 is a perspective view showing an attachment 13 according to the embodiment. Fig. 10 is a side view showing an aspect of usage of the attachment 13. Fig. 11 is a perspective view showing an aspect of usage of the attachment 13.

A light-shielding member 33 of the attachment 13 is formed as a stretchable covering member having a light-shielding property and covering the support portion 2. The light-shielding member 33 is formed by which a flexible and stretchable sheet having a light-shielding property is formed into a cylindrical shape. For example, the light-shielding member 33 is formed of silicone rubber. In a natural state in which no external force is applied to the light-shielding member 33, an opening diameter of at least a distal end part 33a is smaller than a diameter of a distal end surface of the endoscope insertion portion 101. The light-shielding member 33 has a degree of size and stretchablity such that the support portion 2 and the endoscope insertion portion 101 can be inserted therethrough. The light-shielding member 33 is attached to the outer peripheral surface side of the side wall portion 2a of the support portion 2 to be movable in the longitudinal axis direction relative to the support portion 2.

As shown in Fig. 9, a plurality of drawing yarns (operation members) 43 which are separated from each other in a circumferential direction of the distal end part 33a are fixed to an inner peripheral surface of the distal end part 33a of the light-shielding member 33. The drawing yarn 43 is provided to extend inside the light-shielding member 33 and the side wall portion 2a. When the attachment 13 is mounted on the endoscope insertion portion 101, the drawing yarn 43 is inserted through the treatment instrument channel 103 to extend to the operation portion side. A knot portion 43a is provided at the proximal end side of each drawing yarn 43, and the knot portions 43a exposed at the operation portion side are configured to be drawn by an operator.

A returning yarn (an operation member) 44 is provided at a proximal end part 33b of the light-shielding member 33. Specifically, a through hole 33c is formed at the proximal end part 33b of the light-shielding member 33 and the returning yarn 44 is inserted into the through hole 33c to be connected thereto so that the returning yarn 44 is attached to the light-shielding member 33. As shown in Fig. 10, when the attachment 13 is mounted on the endoscope insertion portion 101, the returning yarn 44 is disposed in the vicinity of the outer periphery of the endoscope insertion portion 101, is inserted through an external channel 107 which is mounted on the endoscope insertion portion 101, and is exposed at the operation portion side. A knot portion 44a is provided at the proximal end side of the returning yarn 44, and the knot portion 44a exposed at the operation portion side is configured to be pulled by the operator.

An aspect of usage of the attachment 13 will be described. Fig. 10 is a side view showing a light-shielding-released state in which the attachment 13 is mounted on the endoscope insertion portion 101. A state shown in Fig. 10 is an initial state of a procedure using the attachment 13. That is, the support portion 2 is mounted on the endoscope insertion portion 101 and the light-shielding member 33 is disposed such that the distal end part 33a covers the proximal end 2c of the support portion 2 and the proximal end part 33b covers the outer peripheral surface of the distal end part 101a of the endoscope insertion portion 101. Since the side wall portion 2a is configured to transmit light therethrough, a wide field of view of the image-capturing unit of the endoscope apparatus 100 is ensured when the distal end part 33a of the light-shielding member 33 is disposed near the proximal end 2c of the support portion 2 in the initial state (the light-shielding-released state).

Since the light-shielding member 33 is formed as a stretchable member, the light-shielding member comes into contact with the outer peripheral surface of the support portion 2 and the outer peripheral surface of the endoscope insertion portion 101, and is deformed according to the shapes thereof. In the state shown in Fig. 10, the endoscope insertion portion 101 is inserted into a body, but a position of the light-shielding member 33 in an axial direction is immovably maintained due to friction with respect to the outer peripheral surface of the support portion 2 and the outer peripheral surface of the endoscope insertion portion 101. The light-shielding member 33 has friction in which the light-shielding member 33 is slidable on the outer peripheral surface of the support portion 2 and the outer peripheral surface of the endoscope insertion portion 101 when the drawing yarn 43 or the returning yarn 44 is pulled in a longitudinal direction.

First, the operator inserts the endoscope insertion portion 101 in the initial state shown in Fig. 10 into a vicinity of a treatment target site inside a body and determines a marking position around a treatment target site. Subsequently, when the operator pulls all of the knot portions 43a of the drawing yarns 43 toward the proximal end side, the light-shielding member 33 is pulled in a distal direction along the support portion 2 so that the side wall portion 2a is covered. When the operator further pulls the knot portion 43a of the drawing yarn 43, the distal end part 33a of the light-shielding member 33 reaches the distal end part 2b of the support portion 2 and the distal end part 33a contracts inward in a radial direction of the distal end part 2b of the support portion 2 due to an elastic force of the light-shielding member 33. As a result, the opening diameter of the distal end part 33a of the light-shielding member 33 becomes smaller than the outer diameters of the support portion 2 and the endoscope insertion portion 101.

At this time, when the operator simultaneously pulls all of the drawing yarns 43 with the same force, the distal end part 33a is evenly pulled toward the center of the distal end part 2b of the support portion 2 and an open portion is formed in the vicinity of a central axis of the support portion 2 by the distal end part 33a. Meanwhile, when the force pulling the drawing yarns 43 is changed such that only a part of the plurality of drawing yarns 43 is pulled, a position of the open portion (the distal end part 33a) in a plane orthogonal to the axis of the distal end part 2b of the support portion 2 can be formed at a position offset with respect to the central axis. Further, since the light-shielding member 33 is pulled toward the proximal side when the returning yarn 44 is pulled, the position of the open portion (the position of the distal end 3b) is capable of minutely adjusting by operating the returning yarn 44 and the plurality of drawing yarns 43. In addition, for example, when the returning yarn 44 is integrated at the proximal end side similarly to the first embodiment, the distal end part 33a of the light-shielding member 33 is evenly pulled, and thus the position of the open portion is easily disposed at the center.

Subsequently, the operator further presses the endoscope insertion portion 101 toward the tissue, brings the light-shielding member 33 of the attachment 13 covering the distal end part 2b of the support portion 2 into contact with the tissue, and maximizes the intensity of the light source 102 similarly to the first embodiment. As a result, the light emitted from the light source 102 through the open portion (the distal end) 33a of the light-shielding member 33 is transmitted in a thickness direction of the tissue at the marking position P1. At this time, when the second plane P12 of the tissue is observed by the laparoscope 105 while the light source of the laparoscope 105 is extinguished, the light along a contour shape of the open portion (the distal end) 33a is visible, and thus the marking position P1 is capable of being specified from the second plane P12.

Subsequently, marking process (cauterization) is performed when the electric scalpel 106 is disposed at the marking position P1 while the attachment 13 is in contact with the tissue similarly to the above-described embodiments. The marking process is repeatedly performed at several places around the treatment target site.

After the marking process ends, the operator pulls the returning yarn 44 toward the proximal side to pull the light-shielding member 33 toward the proximal side and to return to the initial state shown in Fig. 10. Subsequently, the operator may perform a treatment on the treatment target site by protruding the treatment instrument toward a position farther than the distal end of the support portion 2.

In this way, since the support portion 2 is covered in a state in which an open portion having an opening diameter smaller than the outer peripheral diameter of the endoscope insertion portion 101 is formed at the distal end part 2b of the support portion 2, the light of the light source 102 is transmitted only from the open portion at a position that is separated from the distal end of the endoscope insertion portion 101 in the distal direction.

The attachment 13 according to the embodiment is possible to show a marking position on an opposite plane (the second plane P12) of a tissue by using the endoscope illumination light source similarly to the above-described embodiments.

According this embodiment, in the light-shielding-released state, the light-transmitting property of the side wall portion 2a of the support portion 2 is ensured and the field of view of the image-capturing unit of the endoscope apparatus 100 is ensured. Meanwhile, in the light-shielded state, the side wall portion 2a is also shielded. Therefore, it is possible to increase luminosity of the open portion 3b by preventing the diffusion light from the light source 102 of the endoscope apparatus 100 from leaking to the outer peripheral side of the side wall portion 2a of the attachment 13. In this embodiment, it is possible to appropriately switch between the light-shielded state and the light-shielding-released state by moving the light-shielding member 33 through the operations of the drawing yarn 43 and the returning yarn 44. Therefore, it is possible to repeatedly perform marking and a series of procedures before and after the marking with a simple method.

In this embodiment, an example in which the support portion 2 is formed in a substantially cylindrical shape similarly to the first embodiment has been described, but the present invention is not limited thereto. For example, as shown in Fig. 12, a guide frame 23d may be provided at the distal end surface of the support portion 2. The guide frame 23d includes a ring portion 23a which is disposed at the center portion and a plurality of connection portions 23c which connect the ring portion 23a with an outer peripheral portion 23b. When the guide frame 23d is provided in the support portion 2, the tissue of the marking position P1 is easily punctured by the puncture needle 106A of the tag-indwelling device while being supported by the guide frame 23d when the tissue of the marking position P1 is punctured by the puncture needle 106A while in contact with the light-shielding member 33 similarly to the first embodiment.

In this embodiment, an example in which the support portion 2 is formed in a cylindrical shape and includes the side wall portion 2a has been described, but the support portion is not limited thereto as long as the light-shielding surface can be held at a position distant from the distal end part of the endoscope insertion portion. For example, the support portion may be formed as a substantially cylindrical frame without the side wall portion 2a.

### [Fourth Embodiment]

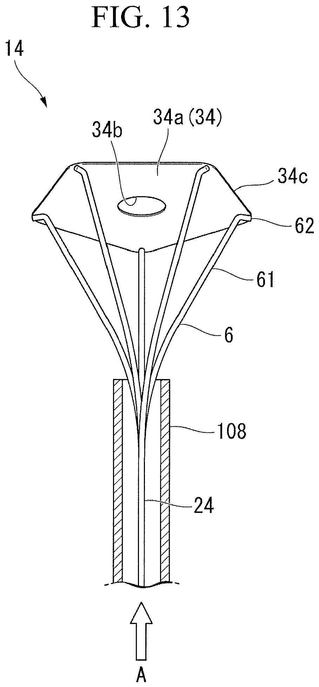

Next, a fourth embodiment of the present invention will be described with reference to Figs. 13 to 15. In this embodiment, configurations of the support portion and the covering member are different from those of the above-described embodiments.

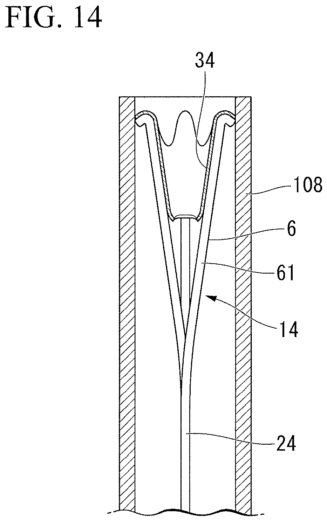

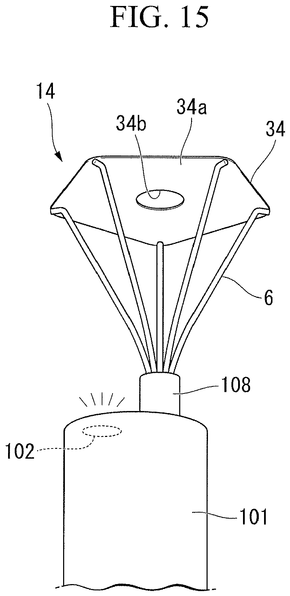

Fig. 13 is a perspective view showing an attachment 14 according to the embodiment. Fig. 14 is a cross-sectional view showing an aspect of usage of the attachment 14. Fig. 15 is a perspective view showing an aspect of usage of the attachment 14.

A support portion 24 of the attachment 14 includes a plurality of elastic linear members 6. An arm portion 61 is provided at a distal end part of the linear member 6. Since the arm portion 61 has a predetermined curved shape, the arm portion is elastically deformed when an external force is applied thereto and is restored to the predetermined curved shape in a natural state. In this embodiment, an example is shown in which five linear members 6 are provided.

As shown in Fig. 13, in the support portion 24, the linear members 6 are integrated and fixed at the proximal side such that distal ends 62 of the plurality of arm portions 61 are arranged to curve and to be away from each other in the natural state and a positional relationship is maintained. When viewed from a longitudinal axis direction (a direction indicated by an arrow A in Fig. 13) at the proximal side of the support portion 24, the arm portions 61 are radially separated in a direction in which angles therebetween are the same.

A proximal end part of the support portion 24 is inserted through the treatment instrument channel 103 of the endoscope insertion portion 101 to be exposed at the operation portion side. Therefore, the proximal end part of the support portion 24 exposed at the operation portion side is configured to be pulled by an operator.

The light-shielding member 34 is formed as a stretchable film having a light-shielding property and having a substantially pentagonal shape in a plan view. The light-shielding member 34 is provided with an open portion 34b is formed at the light-shielding member 34. The open portion 34b has an opening area which is smaller than the area of the distal end surface of the endoscope insertion portion 101 and passes through the film in a thickness direction. An outer peripheral edge 34c of the light-shielding member 34 is fixed to the distal ends 62 of the plurality of arm portions 61. The light-shielding member 34 has a configuration in which a light-shielding surface 34a is formed on a plane including the distal ends 62 of the plurality of arm portions 61 in the natural state.

The attachment 14 is inserted into a sheath 108 from a distal end of the sheath 108 to be capable of advancing and retracting. As shown in Fig. 14, when the attachment 14 is inserted into the sheath 108, the light-shielding member 34 and the plurality of arm portions 61 are accommodated while being deformed along a lumen of the sheath 108.

Next, a procedure using the attachment 14 will be described.

In an initial state, the whole attachment 14 is inserted into the sheath 108 and the sheath 108 is inserted through the treatment instrument channel 103 of the endoscope insertion portion 101. Thus, the initial state is the light-shielding-released state. The operator presses the endoscope insertion portion 101 toward a vicinity of a treatment target site. Here, when the operator operates the proximal end part of the support portion 24 so that the light-shielding member 34 and the plurality of arm portions 61 protrude from the distal end of the sheath 108, the distal ends 62 of the arm portions 61 curve to be away from each other by a restoration force of the linear member 6. At this time, the light-shielding member 34 is widened in a planar shape at a position that is separated from the distal end of the endoscope insertion portion 101. As a result, as shown in Fig. 15, the light-shielding surface 34a is formed to shield the light of the light source 102 in the periphery of the open portion 34b.

A process of pressing the light-shielding surface against the first plane P11 of the tissue, checking the marking position P1 of the second plane P12 of the tissue using the laparoscope 105, and puncturing the tissue using the electric scalpel 106 is similar to that of the above-described embodiments.

In the attachment 14 according to the embodiment, it is possible to show a marking position on an opposite plane (the second plane P12) of the tissue by using the endoscope illumination light source similarly to the above-described embodiments.

In this embodiment, the attachment 14 is used while being inserted through the sheath 108 inserted into the treatment instrument channel 103 of the endoscope insertion portion 101 to be capable of advancing and retracting. Therefore, the attachment 14 is capable of being removing from the endoscope apparatus after the marking process ends. Thus, interference with other procedures is suppressed.

In this embodiment, a configuration in which five arm portions 61 are provided has been described, but the number of the arm portions 61 may be set to a number which can support the light-shielding member 34 so that the light-shielding surface 34a can be formed at the distal side of the light source 102.

### [Fifth Embodiment]

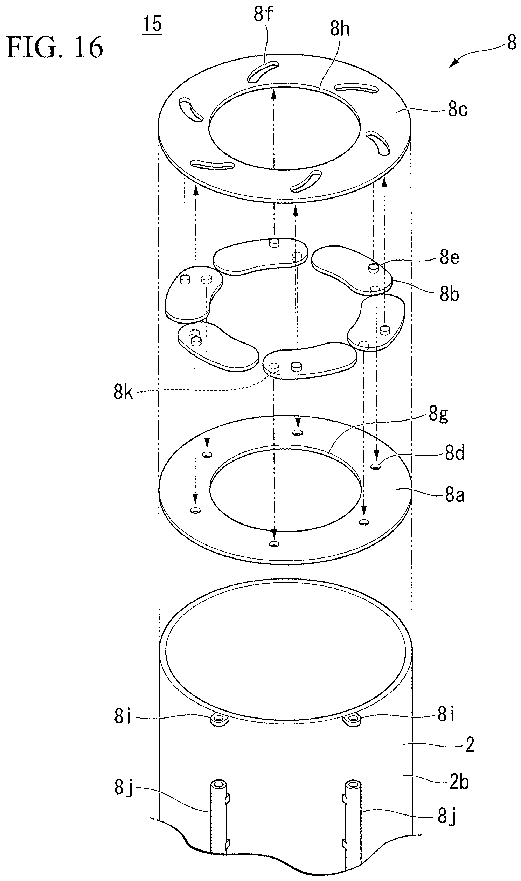

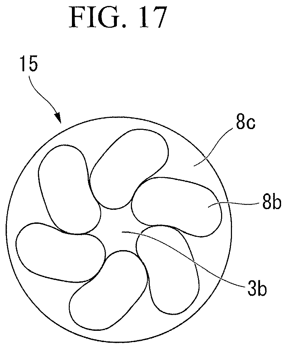

Next, a fifth embodiment of the present invention will be described with reference to Figs. 16 and 17. In this embodiment, a configuration of the light-shielding member is different from those of the above-described embodiments. Fig. 16 is an exploded perspective view showing a light-shielding member 35 of an attachment 15 according to the embodiment. Fig. 17 is a plan view showing a light-shielded state of the attachment.

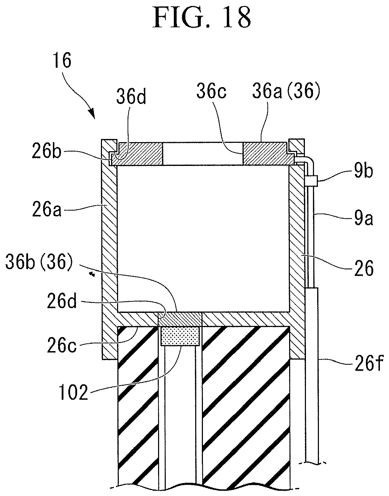

The light-shielding member 35 includes a diaphragm mechanism (a light-shielding area varying mechanism) 8. The diaphragm mechanism 8 has a configuration in which a first member 8a having a ring shape, a plurality of diaphragm blades 8b, and a second member 8c having a ring shape are disposed to be layered in an axial direction in this order of proximity to the support portion 2. Both surfaces of the diaphragm blade 8b are provided with protrusions. Among the protrusions of the diaphragm blades 8b, a protrusion 8k formed on a surface facing the first member 8a is fitted into a hole portion 8d formed in the first member 8a. Among the protrusions of the diaphragm blades 8b, a protrusion 8e formed on a surface facing the second member 8c is disposed to be slidable inside a slide hole 8f formed in the second member 8c. The first member 8a is fixed to the distal end of the support portion 2. The second member 8c is attached to the first member 8a so as to be rotatable in a circumferential direction of the first member 8a. When the second member 8c is rotated relative to the first member 8a, the protrusion 8e of the diaphragm blade 8b slides along the slide hole 8f of the second member 8c. By the sliding of the protrusion 8e, a light-shielding-released state and the light-shielded state are switched between. The light-shielding-released state is a state in which inner open portions 8g and 8h are opened so that the inner open portions 8g and 8h of the first member 8a and the second member 8c are arranged in parallel. The light-shielded state is a state in which the end of the diaphragm blade 8b extends into open portions of the inner open portions 8g and 8h so that an open area is narrowed as shown in Fig. 17. In this embodiment, as shown in Fig. 18, the plurality of diaphragm blades 8b form a light-shielding surface, and an opening formed by the plurality of diaphragm blades 8b forms the open portion 3b.

An outer peripheral edge of the second member 8c is provided with two insertion holes 8i disposed at an interval of 90 degree or more. A middle portion of an operation wire (not shown) is fixed to one position in the vicinity of the distal end part 2b of the side wall portion 2a of the support portion 2, and both ends of a point, where is fixed to the side wall portion 2a, of the operation wire (the operation member) are inserted through the insertion hole 8i and are inserted through two outer sheaths 8j formed at the proximal side to extend to the operation portion. When the operator pulls one of either of the ends of the operation wire extending to the operation portion, the second member 8c moves in a direction that the second member 8c rotates relative to the first member 8a and the open portions of the inner open portions 8g and 8h are narrowed by the diaphragm blades 8b.

In the attachment 15 according to the embodiment, it is possible to show a marking position on an opposite plane (the second plane P12) of a tissue by using the endoscope illumination light source similarly to the above-described embodiments.

The attachment 15 according to the embodiment can adjust an open area of the open portion through which the light of the light source 102 is transmitted.

### [Sixth Embodiment]

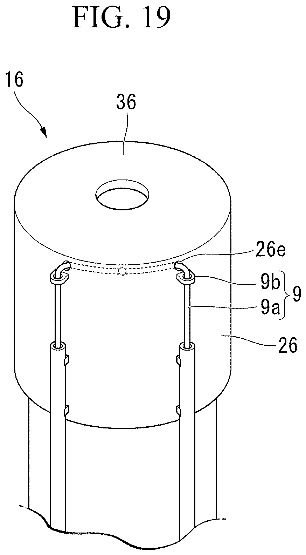

Next, a sixth embodiment of the present invention will be described with reference to Figs. 18 and 19. In this embodiment, the configurations of the light-shielding member and the support portion are different from those of the above-described embodiments. Fig. 18 is a cross-sectional view showing an attachment 16 according to the embodiment. Fig. 19 is a perspective view showing a state in which the attachment 16 is mounted on the endoscope insertion portion 101.

The light-shielding member 36 is provided with, as shown in Fig. 18, a first polarizing filter 36a and a second polarizing filter 36b. The first polarizing filter 36a is attached to a distal end part of a support portion 26. The second polarizing filter 36b is attached to a proximal end part of the support portion 26. In the first polarizing filter 36a, an open portion 36c is formed at the substantial center portion of the first polarizing filter and a step is formed at an outer peripheral edge 36d to be thinner than a body portion. A part between the open portion 36c and the outer peripheral edge 36d is formed as a polarizing filter portion allowing the transmission of light only in a predetermined direction.

The support portion 26 includes a substantially cylindrical side wall portion 26a. A locking groove 26b is formed on an inner peripheral surface of a distal end part of the side wall portion 26a along an entire circumference thereof. The locking groove 26b locks the outer peripheral edge 36d of the first polarizing filter 36a. Since the outer peripheral edge 36d is disposed inside the locking groove 26b, the first polarizing filter 36a is attached to the support portion 26 to be slidable in a circumferential direction of the support portion 26.

The support portion 26 includes an abutting surface 26c formed at a proximal end part thereof. The abutting surface 26c contacts with the distal end surface of the endoscope insertion portion 101 when the attachment 16 is mounted on the endoscope insertion portion 101. The abutting surface 26c is provided with a plurality of open portions. The plurality of open portions is opened to correspond to a plurality of channels open to the distal end surface of the endoscope insertion portion 101. The second polarizing filter 36b is fitted into an opening 26d at a position corresponding to the light source 102 among the plurality of open portions.

The attachment 16 includes a movement mechanism 9. The movement mechanism 9 includes an operation wire (an operation member) 9a and an insertion ring 9b. The insertion ring 9b is provided at two positions of the side wall portion 26a of the support portion 26 at an interval of 90 degree or more. The side wall portion 26a is provided with two communication holes 26e communicating the locking grooves 26b and an outer peripheral surface of the side wall portion 26a. A middle portion of the operation wire 9a is fixed to one point of the outer peripheral edge 36d of the first polarizing filter 36a. Both sides of a point, where is fixed to the first polarizing filter 36a, of the operation wire 9a are disposed at a gap between the locking groove 26b and the outer peripheral edge 36d. Both sides of a point, where is fixed to the first polarizing filter 36a, of the operation wire 9a are respectively inserted through two communication holes 26e to extend to the outside of the side wall portion 26a. The operation wires 9a extending from the two communication holes 26e are respectively inserted through two insertion rings 9b of the side wall portion 26a and are respectively inserted through two outer sheaths 26f provided at the proximal side of the side wall portion 26a to extend to the operation portion.

When the operator pulls one of either of the ends of the operation wire 9a extending to the operation portion, a fixed point between the operation wire 9a and the outer peripheral edge 36d of the first polarizing filter 36a moves in a direction approaching one of the insertion rings 9b and the first polarizing filter 36a moves in the circumferential direction of the support portion 26. In an initial state, a light-shielding-released state is set. The light-shielding-released state is a state that a polarizing direction of the first polarizing filter 36a is aligned to a direction parallel to a polarizing direction of the second polarizing filter 36b so that the transmission of light of the light source 102 is allowed. In the light-shielding-released state, when the operation wire is pulled so that the first polarizing filter 36a is moved to a position where an angle formed between the polarizing direction of the first polarizing filter 36a and the polarizing direction of the second polarizing filter 36b becomes 90 degree, a state is switched to a light-shielded state in which a portion other than the open portion 36c of the first polarizing filter 36a becomes a light-shielding surface which shields the light of the light source 102. When the operator slides the first polarizing filter 36a by pulling the end part of the operation wire 9a so that the polarizing direction of the first polarizing filter 36a becomes parallel to the polarizing direction of the second polarizing filter 36b, a state becomes the light-shielding-released state in which the light emitted from the light source 102 is transmitted through the entire surface of the first polarizing filter 36a, again.

In the attachment 16 according to the embodiment, it is possible to show a marking position on an opposite plane (the second plane P12) of a tissue by using the endoscope illumination light source similarly to the above-described embodiments.

Since the attachment 16 according to the embodiment includes the movement mechanism 9, it is possible to easily change the light-shielded state of the light-shielding surface by the operation of the operation wire 9a.

In this embodiment, a configuration in which the outer sheath 26f through which the operation wire 9a is inserted is provided has been described, but the present invention is not limited thereto. For example, a configuration in which an insertion hole is formed as a through hole communicating the inner and outer peripheral surfaces of the side wall portion 26a, the insertion hole is formed at two positions that are away from each other by 90 degree or more, and both ends of a fixed point of the operation wire 9a are inserted into the support portion 26 from the outside of the through hole to be inserted into the treatment instrument channel 103 similarly to the first embodiment may be employed.

Although the embodiments of the present invention have been described in detail with reference to the drawings, the detailed configuration is not limited to these embodiments and also includes a design change that does not deviate from the gist of the present invention.

In addition, the components shown in each of the above-described embodiments and modified examples can be appropriately used in combination.

### [Industrial Applicability]

It is possible to provide an endoscope attachment capable of suitably marking a specific position of a luminal organ so that a mark is visible from the inside and the outside of the luminal organ.

### [Reference Signs List]

1, 12, 13, 14, 15, 16 Endoscope attachment
2, 22, 24, 26 Support portion
2a Side wall portion (light-transmitting portion)
3, 32, 33, 34, 35, 36 Light-shielding member
8 Diaphragm mechanism (light-shielding area varying mechanism)
9 Movement mechanism
9a Operation wire (operation member)
3a, 34a Light-shielding surface
3b, 36c Open portion
33a Distal end (open portion)
61 Arm portion
36a First polarizing filter
36b Second polarizing filter
41 First yarn (operation member)
42 Second yarn (operation member)
43 Drawing yarn (operation member)
44 Returning yarn (operation member)

## Claims

1. An endoscope attachment comprising:
a light-shielding member disposed in a distal direction from a distal end of an endoscope insertion portion of an endoscope and shields a part of light emitted from a light source of the endoscope at a position distally apart from the light source; and
a support portion connected to the endoscope insertion portion and being capable of holding the light-shielding member at a position distant from the distal end of the endoscope insertion portion.

2. The endoscope attachment according to Claim 1, further comprising:
an operation member connected to the light-shielding member, provided to extend to an operation portion of the endoscope, and switches between a light-shielded state and a light-shielding-released state by moving the light-shielding member, the light-shielded state in which the light-shielding member is disposed at a position on a distal extension line of the light source, the light-shielding-released state in which the light-shielded state is released.

3. The endoscope attachment according to Claim 1 or 2,
wherein the light-shielding member includes a light-shielding surface covering a distal end surface of the endoscope insertion portion at a position distant from the distal end surface of the endoscope insertion portion, and
wherein an open portion transmitting light in an optical axis direction of the light source is formed at a part of the light-shielding surface.

4. The endoscope attachment according to any one of Claims 1 to 3,
wherein the support portion is a cylindrical member being fittable onto the distal end part of the endoscope insertion portion.

5. The endoscope attachment according to Claim 4,
wherein the light-shielding member is a lid-shaped member disposed at a distal end of the support portion, and
wherein the light-shielding member is provided to be detachable from the distal end of the support portion.

6. The endoscope attachment according to Claim 4 or 5,
wherein the support portion includes a light-transmitting portion, which is capable of transmitting light, on a side surface parallel to a longitudinal axis.

7. The endoscope attachment according to Claim 3,
wherein the light-shielding member is formed by a stretchable covering member having a light-shielding property and capable of covering the support portion, and
wherein the light-shielding member covers the support portion so as to be capable to forming an open portion having an opening diameter smaller than an outer peripheral diameter of the endoscope insertion portion at a distal end of the support portion and the light-shielding member makes transmission of light only from the open portion at a position distant from the distal end of the endoscope insertion portion.

8. The endoscope attachment according to Claim 1 or 2,
wherein the support portion includes a plurality of elastic arm portions having a restoration force to restore each of the arm portions to a predetermined curved shape,
wherein the light-shielding member is formed by a stretchable film having a light-shielding property, the light-shielding member is configured such that:
an open portion having an area smaller than that of a distal end surface of the endoscope insertion portion is formed;
an outer peripheral edge is fixed to distal ends of the plurality of arm portions; and
a light-shielding surface is capable of being formed on a plane including the distal ends of the plurality of arm portions,
wherein the light-shielding member and the plurality of arm portions are formed in a deformable manner to be inserted into a sheath, which is inserted through a channel formed along a longitudinal axis direction of the endoscope insertion portion, and to be capable of advancing and retracting from a distal end of the sheath, and
wherein the distal ends of the arm portions are configured to curve to be away from each other when the light-shielding member and the arm portions protrude from the distal end of the sheath, and the light-shielding member forms the light-shielding surface at a position distant from the distal end of the endoscope insertion portion and shields light in a periphery of the open portion.

9. The endoscope attachment according to Claim 3,
wherein the light-shielding member is a lid-shaped member disposed at a distal end of the support portion and is provided with the open portion, and
wherein the light-shielding member includes a light-shielding area varying mechanism that is capable of changing a size of a light-shielding area in a periphery of the open portion.

10. The endoscope attachment according to Claim 3,
wherein the light-shielding member includes:
a first polarizing filter disposed at a distal end of the support portion and provided with the open portion,
a second polarizing filter disposed adjacent to the light source and at a proximal end of the support portion, and
a movement mechanism that rotates the first polarizing filter in a circumferential direction of the support portion, and
wherein the light-shielding member is configured to be capable of shielding in a periphery of the open portion by the movement mechanism that changes a polarizing angle with respect to the second polarizing filter by rotating the first polarizing filter.
